# EUROPEAN PATENT APPLICATION

(11) **EP 2 746 251 A2**
(43) Date of publication of application: **25.06.2014**
(21) Application number: 14153622.7
(22) Date of filing: 03.09.2006
(51) Int. Cl.: C07C 233/25, A61K 31/167, A61K 31/22, A61P 29/00, A61P 11/06, A61P 35/00, A61P 27/06, A61P 27/16, A61P 17/00

(54) **Positively charged water-soluble prodrugs of acetaminophen and related compunds with very fast skin penetration rate**

(62) Divisional of application: 06795894.2
(71) Applicant: Techfields Biochem Co. Ltd, Shanghai 200444 (CN); Yu, Chongxi, Plainfield, Illinois 60585 (US)
(72) Inventor: Yu, Chongxi, Plainfield, IL Illinois 60585 (US); Xu, Lina, 200444 Shanghai N/A (CN)
(74) Representative: Viering, Jentschura & Partner Patent- und Rechtsanwälte

(57) **Abstract**

The novel positively charged pro-drugs of acetaminophen, acetaminosalol, and related compounds in the general formula (1) 'Structure 1' were designed and synthesized. The positively charged amino groups of these pro-drugs not only largely increases the solubility of the drugs, but also bonds to the negative charge on the phosphate head group of membranes and pushes the pro-drug into the cytosol. The results suggest that the pro-drugs diffuses through human skin ∼150 times faster than does acetaminophen, acetaminosalol, and related compounds. It takes 1-2 hours for acetaminophen and acetaminosalol, and related compounds to reach the peak plasma level when they are taken orally, but these prodrugs only took about ∼50 minutes to reach the peak plasma level when they are taken transdermally. In plasma, more than 90% of these pro-drugs can be changed back to the parent drugs in a few minutes. The prodrugs can be used medicinally for treating any NSAIAs-treatable conditions in humans or animals. The prodrugs can be administered not only orally, but also transdermally for any kind of medical treatments and avoid most of the side effects of NSAIAs. Controlled transdermal administration systems of the prodrugs enables acetaminophen, acetaminosalol, and related compounds to reach constantly optimal therapeutic blood levels to increase effectiveness and reduce the side effects of acetaminophen, acetaminosalol, and related compounds. Another great benefit of transdermal administration of these pro-drugs is that administering medication, especially to children, will be much easier.

## Description

### Technical Field

The present invention relates to the preparations of positively charged and watersoluble pro-drugs of acetaminophen, acetaminosalol, and related compounds and their medicinal use in treating any acetaminophen and acetaminosalol-treatable conditions in humans or animals. More specifically, the present invention is to overcome the side effects that are associated with the use of acetaminophen and related compounds. These pro-drugs can be administered orally or transdermally.

### Background Art

N-Acetyl-p-aminophenol (acetaminophen), 4-acetamidophenyl salicylate (acetaminosalol) and related compounds are members of 4-aminophenol group of non-steroidal anti-inflammatory drugs. N-Acetyl-p-aminophenol (acetaminophen) is the leading analgesic and antipyretic drug. Acetaminophen is well tolerated, lacks many of the side effects of aspirin, and is available without prescription. They are used for the relief of signs and symptoms of rheumatoid arthritis and osteoarthritis and for relief of fever.

Unfortunately, a number of side effects are associated with the use of acetaminophen and related compounds, most notably hepatotoxicity and in rare cases nephrotoxicity in humans and in experimental animals. Acute overdosage of acetaminophen results in dose-dependent and potentially fatal hepatic necrosis as well as in rare cases renal tubular necrosis and hypoglycemia. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Fishman and many others (Van Engelen et al. U.S. Pat. No. 6,416,772; Macrides et al. U.S. Pat. No. 6,346,278; Kirby et al. U.S. Pat. No. 6,444,234, Roentsch, et al., U.S. Pat. No. 5,654,337, Park, et al., U.S. Pat. No. 6,190,690, Pearson et al. U.S. Pat. No. 6,528,040, and Botknecht et al. U.S. Pat. No. 5,885,597) have tried to develop a delivery system for transdermal application by formulation. It is very difficult, however, to deliver therapeutically effective plasma levels of these kind drugs into the host by formulation, due to the slow skin penetration rate. Susan Milosovich, et al. designed and prepared testosteronyl-4-dimethylaminobutyrate.HCl (TSBH), which has a lipophilic portion and a tertiary amine groups that exists in the protonated form at physiological pH. They found that the prodrug (TSBH) diffuses through human skin ∼60 times faster than does the drug (TS) itself [Susan Milosovich, et al., J. Pharm. Sci., 82, 227(1993).

### Disclosure of Invention

### Technical Problem

N-Acetyl-p-aminophenol (acetaminophen), 4-acetamidophenyl salicylate (acetaminosalol) and related compounds are members of 4-aminophenol group of non-steroidal anti-inflammatory drugs. N-Acetyl-p-aminophenol (acetaminophen) is the leading analgesic and antipyretic drug. They are used for the relief of signs and symptoms of rheumatoid arthritis and osteoarthritis and for relief of fever.

Unfortunately, a number of side effects are associated with the use of acetaminophen and related compounds, most notably hepatotoxicity and in rare cases nephrotoxicity in humans and in experimental animals. Acute overdosage of acetaminophen results in dose-dependent and potentially fatal hepatic necrosis as well as in rare cases renal tubular necrosis and hypoglycemia.

### Technical Solution

This invention relates to the preparation of novel positively charged pro-drugs of acetaminophen and related compounds and their use medicinally. The pro-drugs of acetaminophen have the general formula (1) 'Structure 1'.

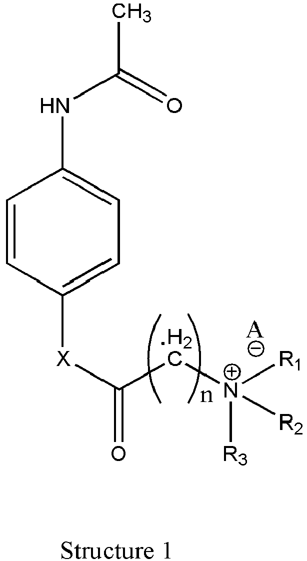

In structure 1, R₁ represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, or 2-OCO-C₆H₄-O; A⁻ represents Cl⁻, Br⁻, F⁻, I⁻, AcO⁻, citrate, or any negative ions; and n=0, 1, 2, 3, 4, 5, 6, 7, 8, 9, 10 ......; All R groups may include C, H, O, S, or N atoms and may have single, double, and treble bonds. Any CH₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of membrane and very hydrophobic drugs will stay in the hydrophobic layer as part of the membrane due to their similarities and cannot enter the cytosol on the inside efficiently.

The goal of this invention is to avoid the side effects of acetaminophen and related compounds by increasing the their solubility in gastric juice and their penetration rate through the membrane and skin barrier which will make it administrable transdermally (topical application). These novel pro-drugs have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs. The solubility of N-Acetyl-4-aminophenyl dimethylaminobutyrate.HCl, 4-acetamidophenyl salicylyl dimethylaminobutyrate.HCl, N-Acetyl-4-aminophenol (acetaminophen), 4-acetamidophenyl salicylate (acetaminosalol) in water are >400 mg, >400 mg, <0.2 mg, <0.1 mg/ml. In many instances, the lowest or rate-limiting step in the sequence is the dissolution of the drug. Acetaminophen, acetaminosalol, and related compounds have a very low solubility in gastric juice. When these new pro-drugs are administered orally in a dosage form such as a tablet, capsule, solution, or suspension, they will dissolve in the gastric juice immediately. The positive charge on the amino groups of these pro-drugs will bond to the negative charge on the phosphate head group of membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. The pH of the stomach is 1-3, so the negative charge on the phosphate head group of the membrane of the gastric mucosa is bonded with proton (H⁺). The positive charges of these prodrugs cannot bond to phosphate head group of the gastric mucosa. These prodrugs will not hurt the stomach.

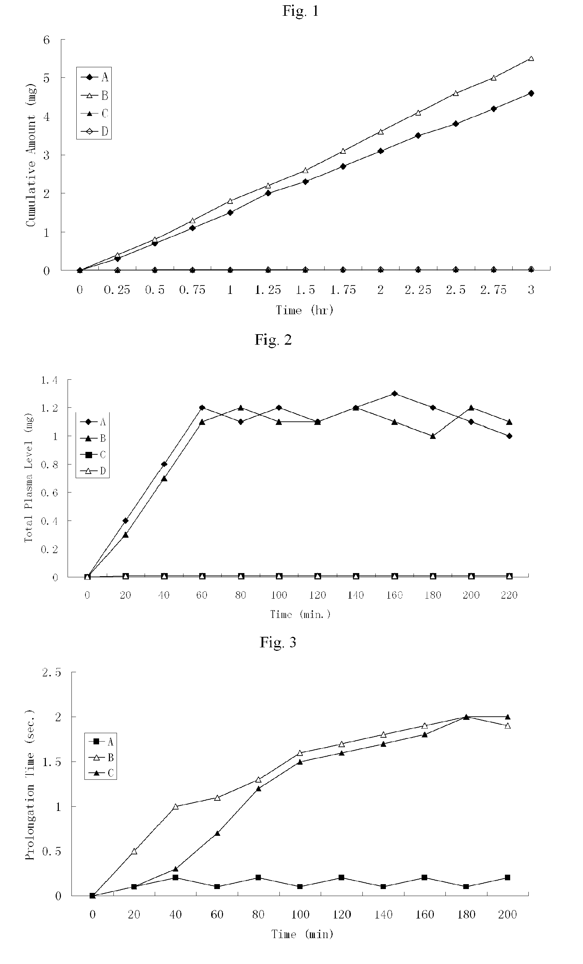

The penetration rates of N-acetyl-p-aminophenyl dimethylaminobutyrate.HCl, 4-acetamidophenyl salicylyl dimethylaminobutyrate.HCl, N-Acetyl-p-aminophenol (acetaminophen), 4-acetamidophenyl salicylate (acetaminosalol) and related compounds through human skin were measured in vitro by using modified Franz cells, which were isolated from human skin tissue (360-400 µm thick) of the anterior and posterior thigh areas. The receiving fluid consisted of 10 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 30% solution of N-Acetyl-p-aminophenyl dimethy-laminobutyrate.HCl and 4-acetamidophenyl salicylyl dimethylaminobutyrate.HCl or a 30% suspension of acetaminophen and acetaminosalol in 2mL of pH 7.4 phosphate buffer (0.2M) are shown in Figure 1. Apparent flux values of 1.5 mg, 1.8 mg, 0.01 mg, and 0.01 mg/cm²/h were calculated for N-acetyl-p-aminophenyl dimethy-laminobutyrate.HCl, 4-acetamidophenyl salicylyl dimethylaminobutyrate.HCl, acetaminophen and acetaminosalol. The results suggest that the positive charge on the di-alkyaminoethyl group has a very important role in the passage of the drug across the membrane and skin barrier. Other prodrugs of the general 'Structure 1' have very high penetration rates and are very close to that of N-acetyl-4-aminophenyl dimethylaminobutyrate.HCl.

The in vivo rates of penetration of N-acetyl-4-aminophenyl dimethylaminobutyrate.HCl, 4-acetamidophenyl salicylyl dimethylaminobutyrate.HCl, acetaminophen and acetaminosalol through the skin of intact hairless mice were compared. The donor consisted of a 20% solution of these compounds in 1 mL of isopropanol applied to a 10 cm² on the backs of the hairless mice. Plasma levels of acetaminophen and 4-acetamidophenyl salicylate were determined by a specific high-performance liquid chromatography method. The results (Figure 2) show that the peak levels of N-acetyl-p-aminophenyl dimethylaminobutyrate.HCl and 4-acetamidophenyl salicylyl dimethylaminobutyrate.HCl were reached in ∼50 minutes after application of the donor systems. It takes 1-2 hours for acetaminophen, acetaminosalol, and related compounds to reach their peak plasma level when they are taken orally. The peak plasma levels were ∼0.01 mg/ml for acetaminophen and acetaminosalol and ∼1.2 mg/ ml for N-acetyl-p-aminophenyl dimethylaminobutyrate.HCl and 4-acetamidophenyl salicylyl dimethylaminobutyrate.HCl (approximately 120 times difference). ∼1.2 mg/ ml of acetaminophen and acetaminosalol in plasma is more than - 50 times higher than plasma level for effective analgesia and effective anti-inflammatory activity. This is a very exciting result. It will be very easy and fast to deliver therapeutically effective plasma level of acetaminophen and acetaminosalol into the host by administration of these prodrugs transdermally. These results suggest that the pro-drugs can be administered not only orally, but also transdermally for any kind of medical treatments. The in vivo rates of penetration of other Pro-drugs of the general 'Structure 1' are close to that of N-acetyl-p-aminophenyl dimethylaminobutyrate.HCl.

The acute toxicity of the prodrugs was investigated. The LD₅₀ orally in mice are: 550 mg/kg, 670 mg/kg, 338 mg/kg, and 550 mg/kg for N-acetyl-p-aminophenyl dimethylaminobutyrate.HCl, 4-acetamidophenyl salicylyl dimethylaminobutyrate.HCl, acetaminophen and acetaminosalol.

Acetaminophen and acetaminosalol demonstrated analgesic and antipyretic activity. A good prodrug should go back to the parent drug in plasma. Diethylaminoethyl ester group of these prodrugs can be rapidly cleaved by the enzymes in human plasma in vitro. More than 90% of the pro-drugs are changed back to their parent drugs. Due to the pro-drugs having a much better absorption rate, the prodrugs will have more strength than their parent drugs at the same dosage. The analgesic and antipyretic activities of these prodrugs were tested using acetaminophen and acetaminosalol as a comparison.

Analgesic activity: The prolongation time of the pain threshold of a mouse tail was determined in accordance with the D'Amour-Smith Method (J. Pharmacol. Exp. Ther., 72, 74(1941)). After 50mg/kg of these prodrugs were administered transdermally, the tails of mice were exposed to heat and the prolongation time of pain threshold was determined. The results obtained are shown in Figure 3. N-acetyl-4-aminophenyl dimethylaminobutyrate.HCl and 4-acetamidophenyl salicylyl dimethylaminobutyrate.HCl have shown analgesic activity nicely.

The number of writhings that occurred when mice were administered an acetic acid solution intraperitoneally were counted, and the rate of inhibition based on the control group was calculated. N-acetyl-4-aminophenyl dimethylaminobutyrate.HCl (100 mg/ kg, B) and 4-acetamidophenyl salicylyl dimethylaminobutyrate.HCl, (100 mg/kg, C) were administered transdermally to the mice 60 minutes before the acetic acid solution was administered. The group A is the control group. The results are shown in Table 1.

**Table 1. The rate of writhings inhibition by prodrugs of acetaminophen and acetaminosalol**

| Group | Dose (mg/kg) | No. of Writhings | % |
|---|---|---|---|
| A | 0 | 35.0 | - |
| B | 100 | 15.6 | 55 |
| C | 100 | 15.7 | 55 |

The results show that the prodrugs demonstrate exceptional analgetic activity. Other compounds of the general 'Structure 1' show similar analgetic activity.

Antipyretic activity: Rats received a sterilized E. coli suspension as a pyrogen. The control group is group A. 2 hours later, N-acetyl-4-aminophenyl dimethylaminobutyrate.HCl (100 mg/kg, B) and 4-acetamidophenyl salicylyl dimethylaminobutyrate.HCl, (100 mg/kg, C) were administered transdermally. The body temperature of rats was taken at 90 min. intervals before and after the administration of the test compounds. The results are shown in Table 2.

**Table 2. Antipyretic Activity of prodrugs of acetaminophen and acetaminosalol**

| Compound | t=0 min. | t=90 min. | t=180 min. | t=270 min. |
|---|---|---|---|---|
| A (Control group) | 37.34±0.05 | 37.36±0.07 | 37.37±0.05 | 37.44±0.08 |
| B (100mg/kg) | 37.32±0.06 | 36.61±0.05 | 36.50±0.08 | 36.50±0.07 |
| C (100mg/kg) | 37.27±0.06 | 36.63±0.05 | 36.52±0.08 | 36.50±0.07 |

The results shown that the prodrugs demonstrated strong antipyretic activity at 100 mg/kg dose. Other compounds of the general 'Structure 1' show similar antipyretic activity.

It is also known that a high oral dose of some of NSAIAs shows an anti-reactive-antiasthmatic activity by inhibition of the cyclooxygenase activity. Due to their very high membrane penetration rate, these prodrugs can be used in treating asthma by spraying into the mouth or nose of the host.

These prodrugs can also be used to treat psoriasis, acne, sunburn or other skin disorders due to inhibition of the cyclooxygenase activity and very high skin penetration rate. They may useful for treating skin, lung, breast, and other cancers.

The present invention relates to pharmaceutical preparations comprising of prodrugs of the general 'Structure 1' in addition to customary auxiliaries and excipients, e.g. in the form of tablets, capsules or solutions for administration orally and in the form of solutions, lotion, ointment, emulsion or gel for transdermal administration. The new active compounds of the general 'Structure 1' can be combined with vitamins such as A, B, C or E or beta-carotene, or other pharmaceuticals, such as beta-carotene, N-acetylcysteine , Caffeine , pseudoephedrine , azapirone , folic acid, etc., for treating any acetaminophen and acetaminosalol-treatable conditions in humans or animals.

Transdermal therapeutic application systems of compounds of the general 'Structure 1' or a composition comprising of at least one compound of the general 'Structure 1', as an active ingredient, can be used for treating any acetaminophen and acetaminosalol-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables to reach constantly optimal therapeutic blood levels to increase effectiveness and reduce the side effects of acetaminophen and acetaminosalol. These systems can be worn on the wrist, ankle, arm, leg, or any part of body.

The compounds of the general formula (1) 'Structure 1' indicated above can be prepared from acetaminophen, acetaminosalol, and related compounds, by reaction with compounds of the general formula (2) 'Structure 2' by using coupling reagents, such as N,N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al. 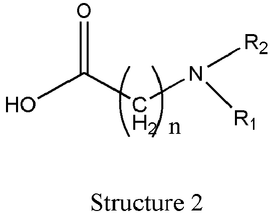 In structure 2, R₁ represents H, one of any alkyl, alkyloxy, alkenyl, or alkynyl residues having 1 to 12 carbon atoms, or aryl residues; R₂ represents H, one of any alkyl, alkyloxy, alkenyl, or alkynyl residues having 1 to 12 carbon atoms, or aryl residues, and n=0, 1, 2, 3, 4, 5, 6, 7, 8, 9, 10 ........

The compounds of the general formula (1) 'Structure 1' indicated above can be prepared from acetaminophen, acetaminosalol, and related compounds, by reaction with compounds of the general formula (3) 'Structure 3'. 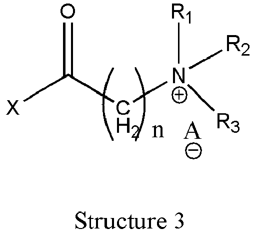 In structure 3, R represents H, one of any alkyl, alkyloxy, alkenyl, or alkynyl residues having 1 to 12 carbon atoms, or aryl residues; R 2 represents H, one of any alkyl, alkyloxy, alkenyl, or alkynyl residues having 1 to 12 carbon atoms, or aryl residues; R₃ represents H, one of any alkyl, alkyloxy, alkenyl, or alkynyl residues having 1 to 12 carbon atoms, or aryl residues; X represents halogen, or p-toluenesulphonyl, A⁻ represents Cl⁻, Br⁻, F, I⁻, AcO⁻, citrate, or any negative ions; and n=0, 1, 2, 3, 4, 5, 6, 7, 8, 9, 10.....

### Advantageous Effects

These pro-drugs of acetaminophen, acetaminosalol, and related compounds have a lipophilic portion and a hydrophilic portion (the amine groups that exist in the protonated form at physiological pH). The positively charged amino groups of these pro-drugs have two major advantages. First, it largely increases the solubility of the drugs; when these new pro-drugs are administered orally in a dosage form such as a tablet, capsule, solution, or suspension, they will dissolve in gastric juice immediately. Second, the positive charge on the amino group of these pro-drugs will bond to the negative charge on the phosphate head group of membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Experiment results show that more than 90% of the pro-drugs were changed back to the parent drugs. The pro-drugs have a much better absorption rate, and thus the prodrugs will have better strength than acetaminophen, acetaminosalol, and related compounds at the same dosage. The experiment results suggest that the pro-drugs, N-acetyl-4-aminophenyl dimethylaminobutyrate.HCl and 4-acetamidophenyl salicylyl dimethylaminobutyrate.HCl diffuses through human skin ∼150 times faster than does acetaminophen and acetaminosalol. It takes 1-2 hours for acetaminophen, acetaminosalol, and related compounds to reach the peak plasma level when they are taken orally, but these pro-drugs only took about ∼50 minutes to reach the peak plasma level. The most exciting result is that the pro-drugs can be administered not only orally, but also transdermally for any type of medical treatment and should avoid most of the side effects of acetaminophen, acetaminosalol, and related compounds, most notably hepatotoxicity and renal toxicity. Another great benefit of transdermal administration of these pro-drugs is that administering medication, especially to children, will be much easier.

### Description of Drawings

Figure 1: Cumulative amounts of N-acetyl-4-aminophenyl dimethylaminobutyrate.HCl (A, 30% solution), 4-acetamidophenyl salicylyl dimethylaminobutyrate.HCl (A, 30% solution), N- acetaminophen (A, 30% suspension), and 4-acetamidophenyl salicylate (D, 30% suspension) crossing isolated human skin tissue in Franz cells (n=5). In each case, the vehicle was pH 7.4 phosphate buffer (0.2 M).
Figure 2: Total plasma levels of acetaminophen and acetaminosalol after topical application of 1 ml of N-acetyl-4-aminophenyl dimethylaminobutyrate.HCl, 4-acetamidophenyl salicylyl dimethylaminobutyrate.HCl, acetaminophen and acetaminosalol in isopropanol to the backs of hairless mice (n=5).
Figure 3: The prolongation time of the pain threshold of mice tails after 50mg/kg of N-acetyl-4-aminophenyl dimethylaminobutyrate.HCl and 4-acetamidophenyl salicylyl dimethylaminobutyrate.HCl were administered transdermally. Group A is the control group.

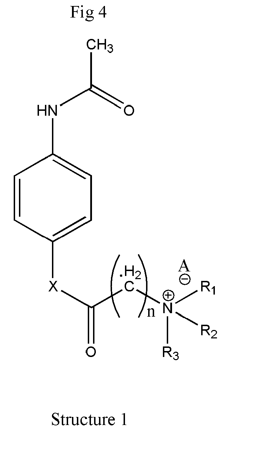

Figure 4. in structure 1, R represents H, one of any alkyl, alkyloxyl, alkenyl or alkynyl residues having 1 to 12 carbon atoms, or aryl residues; R₂ represents H, one of any alkyl, alkyloxy, alkenyl or alkynyl residues having 1 to 12 carbon atoms, or aryl residues; R₃ represents H, one of any alkyl, alkyloxy, alkenyl or alkynyl residues having 1 to 12 carbon atoms, or aryl residues; X represents O, or 2-OCO-C₆H₄ -O; A⁻ represents Cl⁻, Br⁻, F, I⁻, AcO⁻, citrate, or any negative ions; and n=0, 1, 2, 3, 4, 5, 6, 7, 8, 9, 10 ......; All R groups may include C, H, O, S, or N atoms and may have single, double, and treble bonds. Any CH₂ groups may be replaced with O, S, or NH.

### Best Mode

### Preparation of N-acetyl-4-aminophenyl dimethylaminobutyrate.HCl

15.1 g (0.1 mol) of acetaminophen was dissolved in 200 ml of acetone and 200 ml of 10% NaHCO₃. 18.6 g (0.1 mol) of dimethylaminobutyryl chloride hydrochloride was added into the mixture was stirred for 3 hours at RT. The solvents were evaporated off. 500 ml of ethyl acetate was added into the reaction mixture and the mixture was washed with 5% NaHCO₃ (1 x 200 ml) and water (3 x 100 ml). The organic solution was dried over anhydrous sodium sulfate. Sodium sulfate was removed by filtration. HCl gas is bubbled into the solution. The solid product was collected by filtration. After drying, it yielded 26 g of the desired product (86.4%). Hygroscopic product; Solubility in water: 400 mg/ml; Elementary analysis: C₁₄H₂₁ClN₂O₃; MW: 300.78. Calculated % C: 55.90; H: 7.04; Cl: 11.79; N: 9.31; O: 15.96; Found % C: 55.96; H: 7.06; Cl: 11.76; N: 9.29; O: 15.93. ¹H-NMR (400 MHz, D₂O): δ: 1.98 (s, 3H), 2.01 (m, 2H), 2.21 (m, 2H), 2.90 (s, 6H), 3.24 (m, 2H), 7.05 (m, 2H), 7.60 (m, 2H), 7.80 (b, 1H).

### Mode for Invention

### Preparation of N-acetyl-4-aminophenyl diethylaminobutyrate.HCl

15.1 g (0.1 mol) of acetaminophen and 16 g (0.1 mol) of diethylaminobutyric acid were dissolved in 300 ml of dichloromethylene. The mixture is cooled to 0 °C with ice bath. 20.6 g (0.1 mol) of N, N'-Dicyclohexylcarbodiimide was added into the reaction mixture. The mixture was stirred for 1 hour at 0 °C and 2 hours at RT. The solid is removed by filtration. The dichloromethylene solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. The solid product was collected by filtration. After drying, it yielded 27 g of the desired product (82.1 %). Hygroscopic product; Solubility in water: 400 mg/ml; Elementary analysis: C₁₆H₂₅ClN₂O₃; MW: 328.83. Calculated % C: 58.44; H: 7.66; Cl: 10.78; N: 8.52; O: 14.60; Found % C: 58.40; H: 7.68; Cl: 10.76; N: 8.55; O: 14.61. ¹H-NMR (400 MHz, D₂O): δ: 1.50 (t, 6H), 2.00 (m, 2H), 2.02 (s, 3H), 2.21 (m, 2H), 3.24 (m, 2H), 3.27 (m, 4H), 7.05 (m, 2H), 7.60 (m, 2H), 7.80 (b, 1H).

### Preparation of 4-acetamidophenyl salicylyl dimethylaminobutyrate.HCl

27.1 g (0.1 mol) of acetaminosalol was dissolved in 200 ml of acetone and 200 ml of 10% NaHCO₃. 18.6 g (0.1 mol) of dimethylaminobutyryl chloride hydrochloride was added into the mixture was stirred for 3 hours at RT. The solvents were evaporated off. 500 ml of ethyl acetate was added into the reaction mixture and the mixture was washed with 5% NaHCO₃ (1 x 200 ml) and water (3 x 100 ml). The organic solution was dried over anhydrous sodium sulfate. Sodium sulfate was removed by filtration. HCl gas is bubbled into the solution. The solid product was collected by filtration. After drying, it yielded 36 g of the desired product (85.5%). Hygroscopic product; Solubility in water: 400 mg/ml; Elementary analysis: C₂₁H₂₅ClN₂O₅; MW: 420.89. Calculated % C: 59.93; H: 5.99; Cl: 8.42; N: 6.66; O: 19.01; Found % C: 59.96; H: 6.02; Cl: 8.40; N: 6.64; O: 18.98. ¹H-NMR (400 MHz, D₂O): δ: 1.99 (s, 3H), 2.01 (m, 2H), 2.21 (m, 2H), 2.90 (s, 6H), 3.24 (m, 2H), 7.13 (m, 2H), 7.22 (m, 2H), 7.47 (m, 1H), 7.60 (m, 2H), 7.80 (b, 1H), 8.10 (m, 1H).

### Preparation of 4-acetamidophenyl salicylyl dimethylaminobutyrate.HCl

27.1 g (0.1 mol) of acetaminosalol and 16 g (0.1 mol) of diethylaminobutyric acid were dissolved in 300 ml of dichloromethylene. The mixture is cooled to 0 °C with ice bath. 20.6 g (0.1 mol) of N, N'-Dicyclohexylcarbodiimide was added into the reaction mixture. The mixture was stirred for 1 hour at 0 °C and 2 hours at RT. The solid is removed by filtration. The dichloromethylene solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. The solid product was collected by filtration. After drying, it yielded 39 g of the desired product (86.9 %). Hygroscopic product; Solubility in water: 400 mg/ml; Elementary analysis: C₂₃H₂₉ClN₂O₅; MW: 448.94. Calculated % C: 61.53; H: 6.51; Cl: 7.90; N: 6.24; O: 17.82; Found % C: 61.50; H: 6.56; Cl: 7.85; N: 6.22; O: 17.87. ¹H-NMR (400 MHz, D₂O): δ: 1.50 (t, 6H), 2.00 (m, 2H), 2.02 (s, 3H), 2.21 (m, 2H), 3.24 (m, 2H), 3.27 (m, 4H), 7.11 (m, 2H), 7.21 (m, 2H), 7.47 (m, 1H), 7.65 (m, 2H), 7.80 (b, 1H), 8.10 (m, 1H).

### Industrial Applicability

The pro-drugs of the general formula (1) 'Structure 1' are superior to acetaminophen, acetaminosalol, and related compounds. They can be used medicinally in treating any acetaminophen, acetaminosalol, and related compounds-treatable conditions in humans or animals. They may be used for the relief of signs and symptoms of rheumatoid arthritis and osteoarthritis, the reduction of fever, and the treatment of dysmenorrhea. Due to their very high membrane penetration rate, these pro-drugs can be used in treating asthma by inhalation to a host. They can be used to treat psoriasis, acne, sunburn or other skin disorders due to their anti-inflammatory properties. They may useful for treating skin, lung, breast, and other cancers.

### Sequence List Text

Further preferred embodiments are given in the following paragraphs:
[1] The compounds of the general formula (1) 'Structure 1' 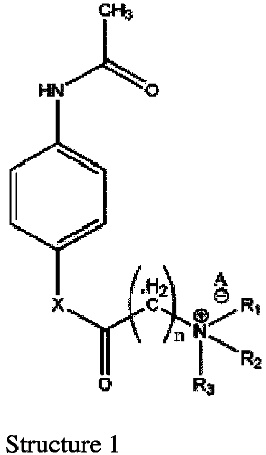 In structure 1, R represents H, one of any alkyl, alkyloxyl, alkenyl or alkynyl residues having 1 to 12 carbon atoms, or aryl residues; R represents H, one of any alkyl, alkyloxy, alkenyl or alkynyl residues having 1 to 12 carbon atoms, or aryl residues; R represents H, one of any alkyl, alkyloxy, alkenyl or alkynyl residues having 1 to 12 carbon atoms, or aryl residues; X represents O, or 2-OCO-C 6 H 4 -O; A" represents Cl", Br", F", I", AcO, citrate, or any negative ions; and n=0, 1, 2, 3, 4, 5, 6, 7, 8, 9, 10 ; All R groups may include C, H, O, S, or N atoms and may have single, double, and treble bonds. Any CH groups may be replaced with O, S, or NH.
[2] Processes for the preparation of compounds of the general formula (1) 'Structure I' according to Embodiment 1, wherein the compounds can be prepared from acetaminophen, acetaminosalol, and related compounds, by reaction with compounds of the general formula (2) 'Structure 2' by using coupling reagents, such as N,N'-Dicyclohexylcarbodiimide, N, N'-Diisopropylcarbodiimide, O- (Benzotriazol-1-yl)-N,N,N',N'-tetramethyluronium tetrafluoroborate, O- (Benzotriazol-1-yl)-N,N,N',N'-tetramethyluronium hexafluorophosphate, Ben- zotriazol-1-yl-oxy-tris(dimethylamino)phosphonium hexafluorophosphate, et al. 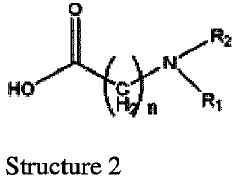 In structure 2, R represents H, one of any alkyl, alkyloxy, alkenyl, or alkynyl residues having 1 to 12 carbon atoms, or aryl residues; R represents H, one of any alkyl, alkyloxy, alkenyl, or alkynyl residues having 1 to 12 carbon atoms, or aryl residues, and n=0, 1, 2, 3, 4, 5, 6, 7, 8, 9, 10 ...
[3] Processes for the preparation of compounds of the general formula (1) 'Structure I' according to Embodiment 1, wherein the compounds can be prepared from metal salts, organic base salts, or immobilized base salts of acetaminophen, ac- etaminosalol, and related compounds, by reaction with compounds of the general formula (3) 'Structure 3'. 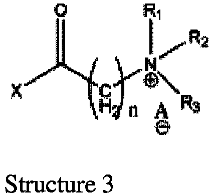 In structure 3, R represents H, one of any alkyl, alkyloxy, alkenyl, or alkynyl residues having 1 to 12 carbon atoms, or aryl residues; R represents H, one of any alkyl, alkyloxy, alkenyl, or alkynyl residues having 1 to 12 carbon atoms, or aryl residues; R represents H, one of any alkyl, alkyloxy, alkenyl, or alkynyl residues having 1 to 12 carbon atoms, or aryl residues; X represents halogen, or p-toluenesulphonyl, A" represents Cl", Br", F, I", AcO, citrate, or any negative ions; and n=0, 1, 2, 3, 4, 5, 6, 7, 8, 9, 10...
[4] Compounds of the general 'Structure 1' or a composition comprising of at least one compound of the general formula (1) 'Structure 1', as an active ingredient, according to Embodiment 1, where they can be administered orally or transdermally, for treating any NSAIAs-treatable conditions in humans or animals. The NSAIAs-treatable conditions include, but are not limited to, pain from a toothache, headache, and arthritis and other inflammatory pain, fever, cancer, dysmenorrhea, radiation-induced vomiting, diabetic neuropathy and acute migraine headache, hemophilic arthropathy, bone loss, and sunburn.
[5] Methods for treating any NSAIAs-treatable conditions in humans or animals by administering transdermally to any part of body (in the from of a solution, spray, lotion, ointment, emulsion or gel) to deliver therapeutically effective plasma levels of the compounds of the general formula (1) 'Structure 1' or a composition comprising of at least one compound of the general formula (1) 'Structure 1', as an active ingredient, according to Embodiment 1.
[6] Methods for topically treating pain such as a headache, toothache, and muscle pain, and arthritis and other inflammatory pain in humans or animals by administering to the inflamed area a therapeutically effective amount of the compounds of the general formula (1) 'Structure 1' or a composition comprising of at least one compound of the general formula (1) 'Structure 1', as an active ingredient, according to Embodiment 1.
[7] Compounds of the general formula (1) 'Structure 1' or a composition comprising of at least one compound of the general formula (1) 'Structure 1', as an active ingredient, according to Embodiment 1, may be administered transdermally, for treating psoriasis, acne, sunburn or other skin disorders in the form of a solution, spray, lotion, ointment, emulsion or gel.
[8] Compounds of the general formula (1) 'Structure 1' or a composition comprising of at least one compound of the general formula (1) 'Structure 1', as an active ingredient, according to Embodiment 1, are administered by spraying to through the mouth or nose or other parts of body for treating asthma.
[9] Compounds of the general formula (1) 'Structure 1' or a composition comprising of at least one compound of the general formula (1) 'Structure 1', as an active ingredient, according to Embodiment 1, for treating any eye inflammatory diseases, for treating of ocular pain after corneal surgery, for treating glaucoma or for treating ear inflammatory and/or painful conditions (otitis) in humans or animals.
[10] Compounds of the general formula (1) 'Structure 1' or a composition comprising of at least one compound of the general formula (1) 'Structure 1', as an active ingredient, according to Embodiment 1, for treating breast cancer, colorestal cancer, pancreatic cancer, skin cancer, and any other cancers.
[11] Transdermal therapeutic application systems of Compounds of the general formula (1) 'Structure 1' or a composition comprising of at least one compound of the general formula (1) 'Structure 1', as an active ingredient, according to embodiment Embodiment 1, for treating any NSAIAs-treatable conditions in humans or animals. These systems can be a bandage or a patch comprising of one active substance- containing matrix layer and an impermeable backing layer. The most preferable system is an active substance reservoir, which has a permeable bottom facing the skin. By controlling the rate of release, this system enables the NSAIAs to reach constantly optimal therapeutic blood levels to increase effectiveness and reduce the side effects of NSAIAs.

## Claims

1. A compound having a general formula of Structure 1: 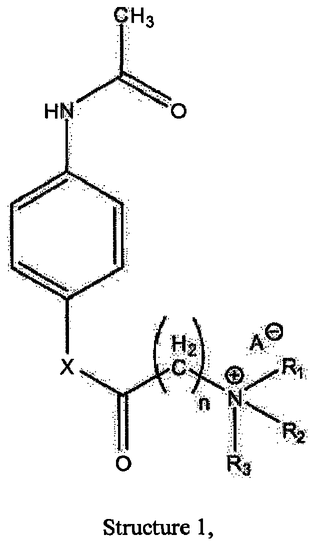 wherein:
R₁ is selected from the group consisting of H, alkyl residues having 1 to 12 carbon atoms, alkyloxy residues having 1 to 12 carbon atoms, alkenyl residues having 1 to 12 carbon atoms, alkynyl residues having 1 to 12 carbon atoms, and aryl residues;
R₂ is selected from the group consisting of H, alkyl residues having 1 to 12 carbon atoms, alkyloxy residues having 1 to 12 carbon atoms, alkenyl residues having 1 to 12 carbon atoms, alkynyl residues having 1 to 12 carbon atoms, and aryl residues;
R₃ is selected from the group consisting of H, alkyl residues having 1 to 12 carbon atoms, alkyloxy residues having 1 to 12 carbon atoms, alkenyl residues having 1 to 12 carbon atoms, alkynyl residues having 1 to 12 carbon atoms, and aryl residues;
any CH₂ of R₁, R₂, and R₃ may be replaced by O, S, or NH;
X represents O, or 2-OCO-C₆H₄-O;
A⁻ represents Cl⁻, Br⁻, F⁻, AcO⁻, citrate, or any negative ion; and
n= 0, 1, 2, 3, 4, 5, 6, 7, 8, 9, or 10.

2. A process for the preparation of a compound according to claim 1, comprising reacting acetaminophen, acetaminosalol or any related compound with a compound having a general formula of Structure 2 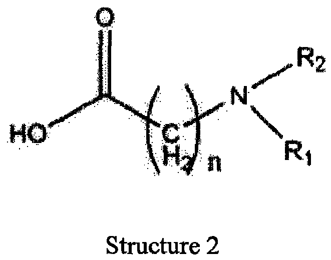 in the presence of a coupling reagent selected from the group consisting of N,N'-dicyclohexylcarbodiimide, N,N'-diisopropylcarbodiimide, O-(benzotriazol-1-yl)-N,N,N',N'-tetramethyluronium tetrafluoroborate, O-(benzotriazol-1-yl)-N,N,N',N'-tetramethyluronium hexafluorophosphate, and benzotriazol-1-yl-oxy-tris(dimethylamino)phosphonium hexafluorophosphate,
wherein R₁, R₂, and n are defined as in claim 1.

3. A process for the preparation of a compound according to claim 1, comprising reacting a metal salt, organic base salt, or an immobilized base salt of acetaminophen, acetaminosalol or any related compound with a compound having a general formula of Structure 3 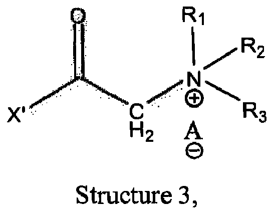 wherein:
R₁, R₂, R₃, A⁻, and n are defined the same as in claim 1; and
X' represents halogen, or p-toluenesulphonyl.
